# EUROPEAN PATENT SPECIFICATION

(11) **EP 2 794 004 B1**
(45) Date of publication and mention of the grant of the patent: **29.07.2020**
(21) Application number: 12801751.4
(22) Date of filing: 17.12.2012
(51) Int. Cl.: A61N 5/06

(54) **IRRADIATION APPARATUS**
BESTRAHLUNGSVORRICHTUNG
APPAREIL D'IRRADIATION

(30) Priority: 19.12.2011 EP 11250931
(43) Date of publication of application: 29.10.2014
(73) Proprietor: Photocure ASA, 0275 Oslo (NO)
(72) Inventor: KLEM, Bjørn, N-0275 Oslo (NO)
(74) Representative: Dehns
(86) International application number: PCT/EP2012/075824
(87) International publication number: WO 2013/092505

(56) References cited:
- WO-A1-2010/078929
- WO-A1-2011/032371
- GB-A- 2 360 461
- ZAWISLAK A ET AL: "Clinical and immunohistochemical assessment of vulval intraepithelial neoplasia following photodynamic therapy using a novel bioadhesive patch-type system loaded with 5-aminolevulinic acid", PHOTODIAGNOSIS AND PHOTODYNAMIC THERAPY, ELSEVIER, AMSTERDAM, NL, vol. 6, no. 1, 1 March 2009 (2009-03-01), pages 28-40, XP026116238, ISSN: 1572-1000, DOI: 10.1016/J.PDPDT.2009.03.004 [retrieved on 2009-05-05]

## Description

This invention relates to an irradiation apparatus for photodynamic therapy of diseases, lesions and conditions of the vulva and/or the anus.

Conditions affecting the vulva include female genital warts and Vulvar Intraepithelial Neoplasia (VIN). The anus can be affected by Anal Intraepithelial Neoplasia (AIN). AIN is similar to VIN and may be treated in a similar fashion.

Female genital warts, VIN and AIN are often caused by persistent human papillomavirus (HPV) infection and are becoming increasingly common in young women. The human papillomavirus (HPV) is a virus that can infect the skin and mucus membranes in humans. More than 100 different types of HPV have been identified. Several HPV types are transmitted through sexual activity and are pathogenic or have oncogenic potential. HPV is estimated to be the most common sexually transmitted infection in the US. Several hundred million women worldwide are infected with HPV once in their life-time (70-80%), with the highest prevalence, 20-30%, occurring in young women 20-25 years of age..

Moderate to severe VINs (VIN2-3) and moderate to severe AIN are referred to as precancerous conditions since if left untreated for a long period cancer can develop. Anal and cervical cancer are a life-threatening diseases. Cervical cancer is today the third most common cancer form among women world wide. Scientists agree that there is a strong correlation between the development of cancers and persistent HPV infections including vulval and anal HPV infections.

No standard treatment for conditions of the vulva such as female genital warts and VIN is available but surgery is being used in the absence of therapeutic options. This often has to be repeated due to high recurrence rates. Multiple surgical treatments can cause vulval disfigurement and loss of sexual function. Alternative treatments include topical application of Imiquimod (Aldara®), an immune response modifier that is applied multiple times per week for several months. However, this treatment is cumbersome due the need for multiple repeated applications, there can be severe adverse reactions and the treatment gave conflicting results (see C.J. Jayne et al., J Reprod Med 2002; 47(5), 395-398).

Photodynamic therapy (PDT) has been used as an experimental treatment for VIN and female genital warts. PDT is a therapeutic modality using a combination of light and a photosensitiser. When illuminated at a suitable wavelength the photosensitiser or "PDT drug" reacts with tissue oxygen to form oxygen radicals that interact with cellular organelles including the mitochondria and cell membranes. These interactions cause cell necrosis or apoptosis (programmed cell death). PDT is today used clinically for the treatment of several diseases, including various skin diseases. Through necrosis and apoptosis of the lesions, PDT will increase the level of antigens and immunological "danger signals" including heat shock proteins. This will stimulate the patient immune system and increase the level of cytotoxic cells (CD8+) that will further contribute to a satisfactory treatment response.

Typical products for use in skin PDT are Metvix® (Galderma, Switzerland) and Levulan® (Dusa Pharmaceuticals Inc, Wilmington, USA).

At present, treatment of VIN with PDT is an investigational procedure (see WO 2010/078929 for example). Most published studies have used local application of 5-aminolevulinic acid (5-ALA). P. L. Martin-Hirsch et al. (Lancet 1998; 351 (9113), 1403) describe the topical use of 5-ALA (20% w/w in Unguentum Merck) VIN patients. After local analgesia and incubation of 5-ALA over 4 hours, the lesions were exposed to non-laser light with a wavelength of 630 nm. Treatment with a light dose of 50 J/cm² showed an unacceptably low response rate while treatment with a light dose of 100 J/cm² showed a clearance rate of 40-60%. P. Hillemanns et al. (Int. J. Cancer; 85 (2000), 649-653) report about 25 patients with 111 lesions of VIN 1-3 who were topically treated with 10 ml 5-ALA solution (20% w/w in saline). After an incubation under occlusion for 4-5 hours, the lesions were exposed to an argon-ion-laser-pumped dye laser providing light at 635 nm. A total light dose of 100 J/cm² was applied, with an irradiance (fluence rate) of 150 mW/cm². Patients were repeatedly treated with PDT and a complete response was achieved in 52% of the patients. A. Zawislak et al. (Photodiagnosis and Photodynamic Therapy (2009), 6, 28-40) describe the topical use of a bioadhesive patch (3x5 cm) which contained a dose of 38 mg/cm² of 5-ALA in dry form. The patch remained *in situ* for 4-6 hours and PDT was carried out with a non-laser lamp with a wavelength of 630 nm over 20-30 min providing a light dose of 100 J/cm². Local anesthesia was available to the patients during and after PDT. Patients were repeatedly treated with PDT. A clearance rate of 52% was achieved.

Such clearance rates are similar to those of laser evaporation or local excision mostly because of the multifocal nature of VIN disease. However, considerably shorter healing times and no scarring was observed.

A range of other photosensitisers than 5-ALA and its derivatives are known from the scientific literature. One type of such compounds is per se toxic to target cells or species or have light emitting properties when exposed to light. Such compounds have a relatively large molecular weight and are often complex molecules like phthalocyanines, chlorines, porphyrins and psoralens. Another, more clinically useful, type of compounds are photosensitiser precursors that per se are not phototoxic, but are converted to photosensitisers, e.g. endogenous porphyrins like protoporphyrin IX (PpIX) in vivo, upon having entered cells. Such compounds are typically 5-ALA and derivatives of 5-ALA like 5-ALA esters, and will be referred to hereafter as "precursors".

Known methods and apparatuses for PDT treatment of the vulva can cause severe pain during photoactivation and for a few days afterwards. Patients are usually either treated after systemic or local analgesia (intravenous opiates or spinal anesthesia) or with repeated interruption of illumination (e.g. pulsed or fractionated illumination). Thus, the treatments can involve subjecting the patient to several hours of treatments, including application of the photosensitising formulation and illumination. The patients will have to visit the gynaecologist to have the formulation applied. Typically, they then have to stay supine for 3-5 hours and then to see the gynaecologist again for illumination. In addition, many of the patients will not respond sufficiently to the first treatment and will therefore have to go through the procedure one or several times.

PDT of VIN is typically carried out by illumination provided by commercially available, external light sources such as xenon arc lamps (Paterson lamp), LED lamps and lasers. An example of the use of a xenon arc lamp is found in Zawislak et al "Clinical and immuno-histochemical assessment of vulval intraepithelial neoplasia following photodynamic therapy using a novel bioadhesive patch-type system loaded with 5-aminolevulinic acid", Photodiagnosis and Photodynamic Therapy, Elsevier, Amsterdam, Netherlands, vol. 6, no. 1, 1 March 2009, pages 28-40.

US 2002/0029071 discloses LED-based PDT probes wherein the LEDs are arranged on the surface of a cylindrical intraluminal probe and on the surface of a spherical head of an intraluminal probe, respectively (Fig. 24 and 25). The probes are for vulval and cervical use. The LEDs give a necessary fluence rate of at least 30 mW/cm² in the red region of the spectrum. The devices are suggested for use in a method of treatment of gynaecological diseases (e.g. VIN). After application of a photosensitiser or a precursor like 5-ALA and incubation thereof, the VIN lesions are exposed to light from the device for 15 to 30 minutes.

As will be appreciated, all of these light sources require operation by a medical practitioner and so will normally be used within a medical institution, such as a hospital or GP surgery. The patient must remain still during the length of the illumination, which is inconvenient and limits the practical length of each treatment session. In addition, the photosensitiser or precursor must be applied to the treatment area prior to use of the apparatus. It is usual with current methods for the patient to wait several hours between application of the photosensitiser/precursor and illumination.

There is hence a need for an improved PDT treatment of VIN and similar intraepithelial neoplasia related to body surfaces, like anal intraepithelial neoplasia (AIN).

According to one aspect the present invention provides a portable, self-contained irradiation apparatus for photodynamic treatment of the vulva and/or the anus, the apparatus comprising: a treatment surface capable of conforming to the area of the vulva and/or anus to be treated, an illumination system for directing light onto a treatment area of the vulva and/or anus, and a power source for the illumination system; wherein the illumination system is arranged to provide light at fluence rates of 50 mW/cm² or below.

A significant advantage of the present invention is that illumination is carried out at very low fluence rates. Fluence rate (or irradiance) refers to the radiant power incident on a unit area and is measured in units of W/cm². Illumination using low fluence rates, i.e. below 50 mW/cm², is known to strongly reduce the patient discomfort (pain) during illumination (see WO 2008/084241 by Photocure ASA) and the following days that require general local or anaesthesia, and may also improve the PDT effect by allowing a continuous build-up of endogenous porphyrins (from precursors) and to prevent oxygen depletion during illumination (S. Jacques et al., "PDT with ALA/PpIX is enhanced by prolonged light exposure putatively by targeting mitochondria", SPIE Proceedings Vol. 2972, "Optical Methods for Tumor Treatment and Detection", ed. T. Dougherty, San Jose, February 1997, and M. Seshadri et al., Clin Cancer Res 14(9), 2796-2805 (2008)).

In preferred embodiments the illumination system is arranged to apply illumination for periods in excess of an hour, preferably for more than two hours, and more preferably for more than four hours, e.g. five hours or six hours. As discussed below, the fluence rate and treatment time may be set based on a desired light dose and based on the severity of the condition to be treated.

The apparatus is more effective due to the use of low fluence rates, preferably applied over a longer period. This apparatus is also more "patient friendly" since it enables treatment of the vulva and/or anus without the patient being required to remain at a medical facility during treatment. The use of the apparatus will often involve only one visit to the medical facility where a suitable apparatus, e.g. in terms of size of the treatment surface may be selected and/or the VIN or AIN lesions may be prepared for the PDT treatment, after which the patient is free to leave. The patient may be able to apply the apparatus themselves. Prolonged ongoing treatment can occur while the patient continues with normal daily activities or overnight, and when the treatment is completed the patient can remove the apparatus for themselves, without the need for a further visit to a medical facility other than a visit to determine treatment success and possible follow-up treatments which are usually necessary but again can be carried out with the apparatus at home by the patient themselves. Thus, the apparatus provides increased comfort and minimises disruption to the patient's other activities.

The energy consumption per unit time of the illumination system should be such that the heating of tissue does not result in undue discomfort or damage to the patient. The irradiation may be applied at a total light dose level of 10 to 200 J/cm², for example at 20 to 150 J/cm² and preferably 30 to 100 J/cm², optionally 20 to 40 J/cm², e.g. 37 J/cm² or 40 J/cm², and this light dose is preferably spread over several hours. The illumination system is arranged to provide, in use, a fluence rate below 50 mW/cm², for example a fluence rate in the range of 0.5 to 40 mW/cm², preferably below 30 mW/cm², and most preferably in the range of 2 to 20 mW/cm², e.g. 5 mW/cm², 6 mW/cm², 8 mW/cm² or 10 mW/cm². This low fluence rate spreads the total light dose over a period of several hours, which can be longer than conventional PDT illumination times as applied in a clinic or similar. As mentioned above this is beneficial both in terms of significantly reduced discomfort for the patient and improved efficacy of the treatment.

The apparatus may include a heat exchanger for removing heat from the treatment surface and/or from the treatment area. The heat exchanger may comprise a thermally conductive path from the treatment surface to a cooler part of the apparatus, for example an opposite surface of the apparatus or to an outer part of the apparatus. The thermally conductive path may comprise a metal, such as copper or aluminium and may advantageously be formed using conductors also used in the electrical connections for the illumination system. The heat exchanger may include or be connected to a heat sink for transfer of heat to the air. The apparatus may be provided with a material for storage of sensible and/or latent heat. A phase change material may be included in thermal conductive relationship with the treatment surface and/or treatment area to remove heat from the treatment area. Preferably the phase change material is a material that changes state at a temperature close to or slightly above normal body temperature. By selecting a phase change material that changes state at a temperature close to or slightly above normal body temperature it is possible to remove large amounts of heat from the treatment area without the user perceiving any increase in temperature.

In a preferred embodiment, however, the apparatus does not include a heat exchanger. With fluence rates below 50 mW/cm², more preferably with fluence rates in the range of 2 to 20 mW/cm², any excess heat which is generated when the apparatus is in use will diffuse into the tissue without causing problems or discomfort.

The illumination system comprises at least one light source, preferably at least one LED and more preferably an array of more than one LEDs or fibre optic light guides, which may be supplied with light by at least one LED. The term "LED" is intended to cover any form of light emitting diode, for example OLEDs (organic light emitting diode) or LECs (light emitting electrochemical cells, as described in A. Sandström et al., Nat. Commun. 3, 2012, 1002). A preferred embodiment comprises an array of 3-80 LEDs, more preferably 10 to 50 LEDs. LEDs are known for providing appropriate wavelengths of light for PDT and can be arranged to provide the low fluence rates used in the apparatus of the invention. The at least one LED may be positioned on or extend out of the treatment surface. In such embodiments it is not necessary for the light to pass through the treatment surface and hence no constraints are placed on its opacity. However, in a preferred embodiment the at least one LED is positioned under the treatment surface in such a way that the light provided by the LEDs passes through the treatment surface onto the area of treatment of the vulva and/or anus.

The wavelength of light used for irradiation may be selected to achieve an efficacious photodynamic effect and hence the LEDs are selected for their ability to emit wavelengths of light suitable for this effect. In one preferred embodiment the at least one LED emits, in use, light having wavelengths in the range of 300 to 800 nm, for example light in the range 500 to 700 nm. In a most preferred embodiment, especially if the apparatus is used together with a composition comprising a precursor selected from 5-ALA or a derivative thereof, e.g. an ester thereof, such wavelengths are used that are readily absorbed by protoporphyrin IX (PpIX), the photosensitiser compound 5-ALA and its derivatives are converted into, when having entered the cells of the tissue which is treated. PplX absorbs at various wavelengths, with a maximum absorption at about 405 nm and further smaller peak absorptions at about 514 nm, 540 nm, 575 nm and 630 nm. Although the maximum absorption is at about 405 nm, light of such wavelength is not preferably used for PDT with 5-ALA or derivatives thereof, since it does not penetrate well into tissue. Hence in a preferred embodiment, in which the apparatus is used together with a composition comprising a precursor selected from 5-ALA or a derivative thereof, e.g. an ester thereof, the illumination system, preferably the at least one LED, is selected to emit light in the range of 600 to 670 nm, preferably light of about 630 nm. The illumination may consist only of the one or more wavelengths or ranges of wavelengths mentioned above.

In some preferred embodiments the illumination system comprises filters to ensure that only light within a certain wavelength range, such as those mentioned above, is emitted from the apparatus. In one embodiment, the treatment surface may be designed to work as a filter, i.e. such that only light having these preferred wavelengths is transmitted. In another preferred embodiment, the illumination system comprises at least one light source, preferably LED which emits a monochromatic light of the desired wavelength, i.e. 630 nm ± 5 nm.

Preferably the apparatus is self-contained in the sense that it is capable of independent operation while the apparatus is applied to the vulva and/or anus. The power source is hence preferably being integrated with the apparatus and arranged to be carried with the apparatus when the treatment surface is, in use, applied to the treatment area of the patient. Advantageously, the use of low fluence rates and LED lighting requires only a small power source, which can hence be easily incorporated into the apparatus without making the apparatus too bulky for comfortable use to treat the vulva and/or anus.

The power source preferably comprises one or more batteries. The batteries should preferably operate via electrochemical reactions using chemicals that are not too toxic for the patient should the apparatus break or leak while in contact with the body. Suitable batteries include lithium batteries e.g. CR2 lithium batteries or equivalents of sufficient capacity which may also be stored for up to 10 years. A flat battery, e.g. of the "coin" type, is preferably used. The slow loss of charge and small size of lithium ion batteries makes them particularly suited for use as the power supply for the apparatus. In one embodiment, the battery is a primary battery. In another embodiment, the battery is a secondary, i.e. rechargeable battery. The latter embodiment is of advantage since VIN/AIN patients usually require multiple, repeated photodynamic treatments in order to achieve full treatment success.

In order to increase the safety of the apparatus, it is preferable that the apparatus includes a housing, preferably a flexible housing, and the power source is sealed within the housing. By sealed it is meant that the housing is fluid tight in use to prevent fluids leaking into or out of the housing. The housing may also include other electrical components, such as components for the illumination system. The housing may comprise two flexible layers sealed to one another about their periphery, with one flexible layer forming the treatment surface. Alternatively the housing may be formed by embedding the power source and/or electrical components within a layer of suitable material, such as a polyurethane layer or silicone layer. This material may be moulded about the power source and/or electrical components to form the housing and optionally to also form the treatment surface. A preferred embodiment comprises a silicone layer encasing the illumination system (e.g. encasing LEDs) with a polyurethane layer on one or both sides of the silicone layer to form a sealed housing.

At its most basic the illumination system can simply comprise electrical connections for the power supply and the at least one LED. With this arrangement the apparatus and thus the illumination system would be activated to switch on the at least one LED when it was desired to begin the treatment. The illumination of the treatment area may continue until the apparatus is switched off and removed, or after a pre-defined and electronically programmed illumination period or until the power supply is depleted.

Activation of the illumination system may be triggered by a switch. In order to allow the power source and other elements of the apparatus enclosed, the switch is preferably enclosed within the apparatus and arranged to be operated whilst sealed within the apparatus. The switch may be a mechanical switch located beneath a flexible part of the apparatus, for example beneath a flexible layer or a flexible moulded part of the housing. Operation of the switch is then permitted by the resilience of the flexible part. Alternatively the switch may be operated by means of an electrical or magnetic field transmitted through the housing. A magnetically operated switch may be implemented by the use of a magnet outside the housing to hold a 'normally closed' reed switch open. When the magnet is removed the reed switch will close and this can be used to activate the illumination system.

In a simple system using just a power source and LED type illumination system it is hard to control the light dose, as the precise life and power output of the power supply will vary. In addition the illumination provided by the at least one LED will be constant. In order to avoid unacceptable heating of tissue, light of low fluence rate is used and it may also be beneficial for the apparatus to be able to provide pulsed light.

Therefore preferably the illumination system of the apparatus according to the invention further comprises a control circuit, such as a microcontroller or microprocessor, for regulating the level of irradiation. The control circuit of the illumination system may be activated by a switch as described above. In a preferred embodiment the control circuit comprises a timer. The illumination system can then be programmed to begin and/or stop illumination at a pre-determined time. For example, in order to allow the absorption and conversion of a precursor into a photosensitiser, e.g. the absorption and build up of porphyrins in case of a precursor such as 5-ALA or derivatives thereof, a certain time is required after application of such a precursor. Hence with the above-discussed timer, it is ensured that sufficient time has passed from application of the precursor to the start of illumination. The length of illumination can also be strictly controlled as the control circuit can be arranged to switch off illumination after a pre-determined time has elapsed which provides the requested light dose. To allow further build-up of endogenous porphyrins from precursors after the first illumination, the apparatus may be programmed to repeat the illumination (re-PDT) after a certain period of time, e.g. 3 hours.

The control circuit may be arranged to provide pulsed illumination. This can be achieved by providing a function generator within a microprocessor. As mentioned above, pulsed light is advantageous in ensuring that no unacceptable heating of tissue occurs. In addition, providing intervals in illumination enhances tissue oxygenation and the effect of PDT. Further it allows for the re-accumulation of endogenous porphyrins in surviving cells that can be treated with repeated illuminations. The frequency and length of the pulses can be chosen according to the requirements of the treatment regime and set within the control circuit.

In one embodiment, the control circuit can be programmed by the user. This enables the length, strength and illumination pattern to be adjusted to suit individual treatments. Suitable re-writable memory forms include EPROM, EEPROM, flash etc. However, the control circuit memory is preferably read only (ROM) and programmed at the time of manufacture.

Access to the control circuit could be achieved by means of a user interface on the apparatus. By answering a series of questions the user can set the initial delay period, dosage duration, number and length of light pulses etc. The interface may be integral with the apparatus. Thus, it may comprise small buttons that may be pressed with a suitable tool or reed switches. Each button or switch may activate a given pre-set condition such as light dose, intensity, pulsed/steady light, etc.

It is preferred that all the electrical components of the illumination system and power source are sealed within the apparatus during use, for example within a housing as discussed above. This improves safety and minimises the possibility of faults caused by fluid incursion. Therefore the control circuit should preferably be sealed within the apparatus.

In some embodiments the user interface may be accessible through a flexible area of the apparatus. Alternatively the apparatus may comprise a sealable opening which provides access to the interface, for example an opening within the housing.

The provision of a user interface however increases the size of the apparatus, which may be undesirable in certain applications. Therefore, alternatively, the control circuit may comprise a receiver for connection to a remote terminal. In this way specific program commands can be communicated from the remote terminal, e.g. a computer, to the control circuit.

In some embodiments the receiver comprises an input port adapted for connection to a cable. In such embodiments the input port is suitably shaped to receive, for example, a USB or other male connector.

The input port should ideally be sealed during use. Therefore the housing may comprise a plug for insertion into the port. The connection comprises seals in order to ensure that the control circuit is sealed within the housing during use.

Alternatively the program commands may be transmitted to the apparatus by means of a wireless connection. For example, the receiver may be an infra-red or radio wave receiver. This has the advantage that a physical input port is not necessary and instead the control circuit can be permanently sealed within the apparatus.

Preferably the control circuit further comprises a feedback system. This enables the control circuit to make adjustments in the treatment program to account for deviations in expected LED performance.

For example, the feedback system may comprise a light monitor or other direct or indirect monitor to measure the light dose that has been given to the patient. In such systems the control circuit may be programmed to switch off the light source, e.g. LED(s), after a pre-determined light dose has been reached rather than a pre-determined time.

Alternatively a dosimeter may override the timer in the event that the light source, e.g. LEDs, do not operate as expected. For example, if the power supply is faulty the output of the LEDs may be reduced. Therefore it will be necessary to continue illumination beyond the pre-determined time in order to obtain a complete light dose. Conversely if the power output of the LEDs is stronger than anticipated the illumination can be stopped ahead of the pre-determined time interval, or the duration of each pulse can be shortened to prevent possible overheating of tissue.

Another optional feature of the control circuit is one or more performance indicator lights for informing the patient whether the apparatus has operated correctly or whether a fault has occurred. The control circuit may be arranged to provide a signal to the patient when treatment is complete to indicate that the apparatus can be removed. For example an acoustic and/or visual signal may be provided, such as an alarm sound and/or a light signal. Alternatively or in addition, a vibration could be used as the signal to indicate the end of the treatment. Typically the patient would be informed of the length of the treatment and so the signal can be used to confirm an expected end of the treatment and hence need not be overly intrusive.

Advantageously, when the control circuit is used to turn off the LEDs at the end of the treatment cycle there is no significant ill effect for the patient if the apparatus remains in place for longer than the treatment time. However, it is expected that patients will wish to know when treatment has ended and the apparatus can be removed.

Preferably some or all of the above mentioned features of the control circuit are contained in a microprocessor.

In preferred embodiments the treatment surface comprises a flexible sheet incorporating the illumination system, for example in the form of LEDs or strips of LEDs or fibre optic light guides, which may be supplied with light by at least one LED and which may be included in a fabric (e.g. Philips Lumalive) or in the form of field-induced polymer electroluminescent lightning (FIPEL) which uses layers of light emitting polymers which contain traces of nanomaterials that glow, when a current is applied. The apparatus may comprise a substrate layer, for example of a flexible plastic, for supporting the illumination system and treatment surface. The treatment surface may be an upper surface formed on or formed integrally with the substrate layer, with the illumination system embedded within the substrate layer beneath the treatment surface. The treatment surface and/or the substrate may be formed of polyurethane or of silicone. The treatment surface is capable of conforming to the area to be treated and thus in contact with the vulva and/or anus, when the apparatus is in use. In a preferred embodiment, the treatment surface is flexible at least to such a degree that such contact is achieved. In another preferred embodiment, the treatment surface includes a protrusion which, when the device is in use, is inserted and located in a body orifice, for example the anus, if the apparatus is used in the treatment of AIN or the vagina, if the apparatus is used in the treatment of VIN. Such a protrusion is preferably of an elongated cylindrical or conical shape and of suitable diameter to be in contact with the walls of the orifice, when located in the orifice. When the apparatus is in use, it extends about 1-3 cm into the orifice, preferably 1-2 cm. When the apparatus is in use, light from the illumination system will pass through the treatment surface and hence also through the protrusion. Hence lesions inside the anus or vagina may be treated concomitantly.

Preferably, the treatment surface is at least partially transparent so as to allow light from the illumination system to pass through the surface to provide the required PDT treatment. In some embodiments the treatment surface may be fully transparent to light having the wavelengths required for PDT treatment and being emitted by the illumination system. However, preferably the material of the treatment surface and/or other material between the treatment surface and the light emitting portion(s) of the illumination system is arranged to diffuse the light, thereby enabling an even distribution of light from multiple LEDs or from multiple fibre optic light guides. In a preferred embodiment diffusing members as disclosed in US 2009/0198173 are used which reduce the number of light sources, e.g. LEDs, for a given area to be illuminated and thus further decrease the heat generated by the apparatus when in use. Preferably, a transparent or translucent silicone is used as a material for the treatment surface which acts as a diffuser for the emitted light. A silicone layer may surround the illumination system (e.g. LEDs) and extend above the light emitting parts of the illumination system. The silicon layer may be placed on top of a polyurethane substrate layer. There may be white pigments within the silicone. The use of white pigments assists in reflecting the light back towards the treated area as well as increasing light diffusion. A protecting polyurethane layer may be placed on top of the silicone layer.

The treatment surface preferably comprises as a top layer a removable and/or cleanable layer, which may for example be a layer located atop the silicone layer or atop a polyurethane layer that is on the silicone. The removable layer can be contacted with the patient's body and removed after use. It may be cleaned or it may be a disposable layer that can be replaced by a new layer for subsequent use of the apparatus. The use of a removable layer in this way permits the apparatus to be re-used for multiple treatments of the same patient or for treatment of different patients, after suitable cleaning. The removable and/or cleanable layer may comprise a polymer foil or film layer. In another preferred embodiment, the treatment surface comprises as a top layer a removable layer which comprises a photosensitiser or precursor, e.g. in the form of a dry composition such as a dry film, preferably such dry compositions comprising 5-ALA or derivatives thereof as described in PCT/EP2011/061688 by Photocure ASA or as described by A. Zawislak et al. in Photodiagnosis and Photodynamic Therapy (2009), 6, 28-40).

The apparatus may comprise a lens system arranged to provide homogenous illumination over the treatment area. The treatment surface may act as the lens system. For example, this surface may be formed of silicone or another material comprising surface elements for diffusing light such as shaped ridges or the like.

The apparatus is preferably arranged to be secured in place with the treatment surface located at the treatment area, such that the patient can continue with normal activities. The apparatus may hence be in the form of a diaper, sanitary napkin, panty liner, or the like, enabling the treatment surface to be comfortably and securely held against the vulva and/or the anus. Any suitable shapes and/or structures can be utilised, for example based on conventional diapers, panty liners, sanitary napkins, sanitary pads, incontinence pads and so on.

The apparatus may be arranged to be incorporated within a garment such as a panty, for example by insertion of the apparatus into a reusable or a disposable garment or by providing the apparatus with an adhesive underside which is suitable to attach it to a garment such as a panty. The apparatus may take the form of a panty or diaper to be worn instead of or within the patient's underwear. The garment may comprise a pouch into which the apparatus can be inserted. Thus, the removable and/or cleanable layer described above may be connected to the garment to form the pouch, with the apparatus being inserted beneath the removable and/or cleanable layer. With this arrangement the removable and/or cleanable layer may be connected to the garment along three sides to form the pouch, accessible by the fourth, open, side.

In preferred embodiments the apparatus includes a coupling for joining the apparatus to the patient or to an undergarment such as a panty. The coupling may comprise a fastener for securing the apparatus about the patient in the manner of a diaper, for example an adhesive or Velcro fastener, or it may be a mechanical fixing such as a button, hook and eye or similar. Alternatively the coupling may comprise a coupling for connection to a garment such as a panty, preferably for connection in or to the gusset or crotch part of the garment. Thus, the coupling may comprise an adhesive patch or patches for adhering the apparatus to the garment, for example an adhesive on an underside of the apparatus, the underside being a surface opposite the treatment surface, or an adhesive on wings at side portions of the apparatus, for connection to a garment in a similar manner to wings on known sanitary pads and the like. In another embodiment, the treatment surface has bioadhesive properties which makes the apparatus stay in place for the time of the treatment (see for instance A. Zawislak et al. in Photodiagnosis and Photodynamic Therapy (2009), 6, 28-40).

Illumination with low fluence rates as used with the present apparatus requires that the illumination will have to occur over a relatively long time period, e.g. many hours, in order to achieve the desired light dose necessary to achieve a therapeutic effect, and hence is very cumbersome in a clinical (hospital) situation. By providing a coupling as described above for securely locating the treatment surface at the treatment area the apparatus permits very long treatment times using the advantageous low fluence rates.

To ensure a comfortable and effective treatment for each patient, the apparatus may be made available in different sizes and/or shapes with a treatment surface of different sizes and/or shapes. Moreover, although the apparatus has been created with the treatment of human patients in mind, it is also possible it to be used in the treatment of other animals. Therefore the shape of the apparatus and/or treatment surface may be dependent on the anatomical structure of the animal on which the apparatus is intended for use.

The treatment surface preferably has a size and or shape selected for complementary fit with the treatment area, and is preferably sized to confront the entire area where PDT is required, for example size to confront the entire area of the vulva or the entire area of the anus or the combined area of the vulva and anus. The illumination system and treatment surface are preferably arranged such that radiation is emitted toward the treatment area at sufficient proximity and intensity to achieve the desired treatment effect.

The apparatus may comprise a handle or tab for gripping the apparatus during application and removal.

The apparatus may be used to provide PDT according to the following method. Firstly a composition comprising a photosensitiser or precursor thereof is topically applied to the area to be treated by the patient or a physician, where applicable by using a specialised applicator. The composition is preferably in the form of a cream, an ointment, a solution, a spray, a powder, a foam or a lotion. Further, it is preferred that the viscosity of the composition is such that the composition stays at the area it is applied to for the time of the treatment, also at the elevated temperatures of the treatment. Alternatively, the area of interest is treated by means of a systematically acting drug. Such a systematically acting drug may be supplied intravenously or orally, for example. Depending on the type of VIN/AIN lesion, it can be necessary that a physician scrapes off the upper keratinized cell layers with a lancet or a ring curette to improve the penetration of a topically applied composition. Prior to application of a topical composition, the area treatment is preferably cleaned to reduce the bacterial flora, e.g. by washing the area with aqueous 0.4% chlorhexidine solution. The apparatus is then applied to the treatment area, either by the patient at home or by a physician in a hospital/private practice. It may be switched on before or after being applied to the treatment area. The patient can then immediately continue their normal daily routine while the treatment area is receiving illumination from the apparatus. In this way treatment can occur over a prolonged period of time without inconvenience to the patient. In a preferred embodiment treatment is carried out overnight, e.g. the patient applies the composition comprising a photosensitiser or precursor thereof to the area to be treated then applies the apparatus to said area and goes to bed. Since a low fluence rate is used, little heat is generated, pain is significantly reduced and the efficacy of the treatment is increased. After the treatment is complete the patient can either return to the medical facility for removal of the apparatus or preferably remove it themselves. The apparatus or parts thereof can either be discarded or returned to the medical facility for disposal.

The apparatus may optionally comprise a drug delivery system to permit the step of topical application of the drug, i.e. composition comprising a photosensitiser or precursor to be done by application of the apparatus to the patient. The drug delivery system may comprise a drug carrying area on the treatment surface, for example on the removable and/or cleanable layer of the treatment surface or the removable layer itself. This might be a textured surface for carrying a composition of photosensitiser or precursor or the treatment surface or removable and/or cleanable layer itself without any further modifications may act as a drug delivering system. The apparatus may automatically perform the illumination either immediately upon application or preferably at a later time.

Optionally the drug delivery system further comprises a physical, mechanical or electrical system related to delivery. Such an optional system may include, for example, filters, membranes, one or more reservoirs arranged to deliver the composition comprising the photosensitiser or precursor based upon a preset plan for drug delivery or based on physical conditions, such as for example pH, osmolality, temperature, pressure, water content in the surroundings. However, the simplest and in most cases the most preferred drug delivery system is just a single drug carrying area for holding the composition, and in a most preferred embodiment, the drug delivery system is the treatment surface or removable and/or cleanable layer itself.

In this preferred embodiment the method of use is similar to that described above except that the composition is not applied to the treatment area in a separate procedure. Instead the composition is applied to the drug carrying area, which may be the treatment surface or a removable and/or cleanable layer, and is hence applied to the body upon application of the treatment surface of the apparatus to the treatment area. Illumination is then conducted as described above.

The composition can be supplied together with the apparatus. In such instances the drug delivery system, i.e. drug carrying area or reservoir, preferably treatment surface, may be supplied with a cover, such as a foil or cap, to seal the composition within the apparatus until use. Prior to use the cover is removed so that the composition can be released. Alternatively the apparatus can be supplied separately from the composition. This enables the physician to choose the optimal composition for a particular case and the physician or patient to add this to the drug delivery system prior to application of the apparatus.

The composition to be used with the apparatus, whether supplied with the apparatus or applied to the apparatus before use or applied to the treatment area separately, may comprise any suitable photosensitiser or precursor of a photosensitiser.

A range of photosensitisers are known in the scientific literature. As discussed above, one type of such compounds are compounds that per se are toxic to target cells or species or have light emitting properties when exposed to light. Such compounds have relatively large molecular weights and are often complex molecules like phthalocyanines, chlorines, porphyrins or psoralens. Another type are compounds that not per se are toxic or light emitting, but form photosensitisers in vivo. Such compounds - referred to herein as precursors - are 5-ALA and derivatives of 5-ALA, like 5-ALA esters. Either type of compound, i.e. photosensitisers or precursors can be used or supplied with the present apparatus.

5-ALA and its derivatives are amongst the most clinically useful precursors of photosensitisers known in the art. These compounds are converted in the body to porphyrins, e.g. protoporphyrin IX (PpIX), which is a photosensitiser that absorbs light and in contact with oxygen generates singlet oxygen. Singlet oxygen is extremely reactive and reacts fast with various cellular biomolecules resulting in cell death.

5-ALA and its derivatives are widely known and used in methods of photodynamic therapy (PDT) for the treatment of various abnormalities or disorders of the skin or other epithelial organs or mucosa, especially cancers or pre-cancerous lesions, as well as certain non-malignant lesions, e.g. skin diseases such as psoriasis, actinic keratosis (AK) and acne. 5-ALA (Levulan®, Dusa) and 5-ALA methyl ester (Metvix®, Galderma, Switzerland) are commercial therapeutic products for PDT treatment of actinic keratosis and basal cell carcinoma.

The use of 5-ALA and derivatives thereof, e.g. 5-ALA esters in PDT is well known in the scientific and patent literature (see, for example, WO 2006/051269, WO 2005/092838, WO 03/011265, WO 02/09690, WO 02/10120 and US 6034267). All such derivatives of 5-ALA and their pharmaceutically acceptable salts are suitable for use with the apparatus herein described and preferably used.

Esters of 5-aminolevulinic acid and N-substituted derivatives thereof are preferred precursors in a composition for use with the invention. Those compounds in which the 5-amino group is unsubstituted, i.e. the ALA esters, are particularly preferred. Such compounds are generally known and described in the literature (see, for example, WO 96/28412 and WO 02/10120 to Photocure ASA).

Esters of 5-aminolevulinic acid with substituted or unsubstituted, preferably substituted, alkanols, i.e. alkyl esters or, more preferably, substituted alkyl esters, are especially preferred precursors in a composition for use with the invention.

Examples of such precursors include those of formula (I) and pharmaceutically acceptable salts thereof:

R²₂N-CH₂COCH₂-CH₂CO-OR¹ (I)

wherein
- R¹: represents a substituted or unsubstituted alkyl group; and
- R²: each independently represents a hydrogen atom or a group R¹

As used herein, the term "alkyl", unless stated otherwise, includes any long or short chain, cyclic, straight-chained or branched saturated or unsaturated aliphatic hydrocarbon group. The unsaturated alkyl groups may be mono- or polyunsaturated and include both alkenyl and alkynyl groups. Unless stated otherwise, such alkyl groups may contain up to 40 carbon atoms. However, alkyl groups containing up to 30 carbon atoms, preferably up to 10, particularly preferably up to 8, especially preferably up to 6 carbon atoms are preferred.

In compounds of formula I, the R¹ groups are substituted or unsubstituted alkyl groups. If R¹ is a substituted alkyl group, one or more substituents are either attached to the alkyl group and/or interrupt the alkyl group. Suitable substituents that are attached to the alkyl group are those selected from: hydroxy, alkoxy, acyloxy, alkoxycarbonyloxy, amino, aryl, nitro, oxo, fluoro, -SR₃, -NR³₂ and -PR³₂, wherein R³ is a hydrogen atom or a C₁₋₆ alkyl group. Suitable substituents that interrupted the alkyl group are those selected from: -O-, -NR₃-, -S- or -PR₃.

If R¹ is a substituted alkyl group, one or more aryl substituents, i.e. aryl groups, preferably one aryl group, are preferred.

As used herein, the term "aryl group" denotes an aromatic group which may or may not contain heteroatoms like nitrogen, oxygen or sulphur. Aryl groups which do not contain heteroatoms are preferred. Preferred aryl groups comprise up to 20 carbon atoms, more preferably up to 12 carbon atoms, for example, 10 or 6 carbon atoms. Preferred embodiments of aryl groups are phenyl and napthyl, especially phenyl. Further, the aryl group may optionally be substituted by one or more, more preferably one or two, substituents. Preferably, the aryl group is substituted at the meta or para position, most preferably the para position. Suitable substituents include halo alkyl, e.g. trifluoromethyl, alkoxy, preferably alkoxy groups containing 1 to 6 carbon atoms, halo, e.g. iodo, bromo, chloro or fluoro, preferably chloro and fluoro, nitro and C₁₋₆ alkyl, preferably C₁₋₄ alkyl. Preferred C₁₋₆ alkyl groups include methyl, isopropyl and t-butyl, particularly methyl. Particularly preferred aryl substituents are chloro and nitro. However, still more preferably the aryl group is unsubstituted.

Preferred such R¹ groups are benzyl, 4-isopropylbenzyl, 4-methylbenzyl, 2-methylbenzyl, 3-methylbenzyl, 4-[t-butyl]benzyl, 4-[trifluoromethyl]benzyl, 4-methoxybenzyl, 3,4-[di-chloro]benzyl, 4-chlorobenzyl, 4-fluorobenzyl, 2-fluorobenzyl, 3-fluorobenzyl, 2,3,4,5,6-pentafluorobenzyl, 3-nitrobenzyl, 4-nitrobenzyl, 2-phenylethyl, 4-phenylbutyl, 3-pyridinyl-methyl, 4-diphenyl-methyl and benzyl-5-[(1-acetyloxyethoxy)-carbonyl]. More preferred such R1 groups are benzyl, 4-isopropylbenzyl, 4-methylbenzyl 4-nitrobenzyl and 4-chlorobenzyl. Most preferred is benzyl.

If R¹ is a substituted alkyl group, one or more oxo substituents are preferred. Preferably, such groups are straight-chained C₄₋₁₂ alkyl groups which are substituted by one, two or three oxo groups. Examples of such groups include 3,6-dioxa-1-octyl and 3,6,9-trioxa-1-decyl.

If R¹ is an unsubstituted alkyl group, R¹ groups that are saturated straight-chained or branched alkyl groups are preferred. If R¹ is a saturated straight-chained alkyl group, C₁₋₁₀ straight-chained alkyl group are preferred. Representative examples of suitable straight-chained alkyl groups include methyl, ethyl, n-propyl, n-butyl, n-pentyl, n-hexyl and n-octyl. Particularly preferred are C₁₋₆ straight-chained alkyl group, most particularly preferred methyl and n-hexyl. If R¹ is a saturated branched alkyl group, such branched alkyl groups preferably consists of a stem of 4 to 8, preferably 5 to 8 straight-chained carbon atoms which is branched by one or more C₁₋₆ alkyl groups, preferably C₁₋₂ alkyl groups. Examples of such saturated branched alkyl groups include 2-methylpentyl, 4-methylpentyl, 1-ethylbutyl and 3,3-dimethyl-1-butyl.

In compounds of formula I, each R² independently represents a hydrogen atom or a group R¹. Particularly preferred for use in the invention are those compounds of formula I in which at least one R² represents a hydrogen atom. In especially preferred compounds each R² represents a hydrogen atom.

The most preferred precursors to be used in a composition together with the devices according to the invention are compounds of formula I and pharmaceutically acceptable salts thereof, wherein R¹ is C₁-C₆ alkyl, e.g. hexyl, more preferably straight chain C₁-C₆ alkyl, e.g. n-hexyl and both R² represent hydrogen, i.e. 5-ALA hexyl ester and pharmaceutically acceptable salts thereof, preferably the HCI salts. The most preferred precursor is 5-ALA hexyl ester and the most preferred pharmaceutically acceptable salt of 5-ALA hexyl ester is the HCI salt.

The composition comprising the photosensitiser or precursor to be used together with the current device can be any type of pharmaceutical formulation and may be prepared by any conventional procedure available in the art (see WO 02/10120 to Photocure ASA). For example, esters of 5-ALA may be prepared by reaction of 5-ALA with the appropriate alcohol in the presence of base. Alternatively compounds for use in the invention may be available commercially (e.g. from Photocure ASA, Norway).

Preferred formulations are liquids (aqueous and non-aqueous), solids such as dusting powder, dry compositions such as films (preferably those dry compositions disclosed in PCT/EP2011/061688), semi-solids such as creams, ointments, gels or pastes (preferred those semi-solids disclosed in WO 2010/142457), foam formulations or other expandable formulations (for example based on heating to body-temperature) and formulations/systems similar to patches. The components in the composition (e.g. excipients and additives) are the same components found in pharmaceutical products on the market, and a listing of such components can be found in handbooks of pharmaceutical excipients.

It is important that the formulation is as such that the composition is absorbed completely into the tissue to be treated or is transparent in order not to interfere with the illumination. As noted above, it is also possible to make use of compositions that are applied systematically, for example drugs that are given to the patient intravenously.

Viewed from another aspect the present invention provides a method of photodynamic therapy of a treatment area on the vulva and/or anus, the method comprising: applying a composition comprising a photosensitiser or precursor to the treatment area; placing an apparatus, preferably an apparatus according to the first aspect, at the treatment area with a treatment surface comprised in said apparatus in contact with said treatment area; and illuminating the treatment area using an illumination system provided in said apparatus; wherein the illumination comprises light at fluence rates of 50 mW/cm² or below.

The fluence rate may be as discussed above in relation to the apparatus of the invention, and the method may comprise the use of an apparatus with any of the features discussed above. The wavelength of the light may be as set out above. The method preferably comprises illuminating the treatment area for a period in excess of an hour, preferably two hours or more and preferably over four hours.

The method may include a step of selecting an apparatus of suitable size and/or shape. The apparatus may be selected firstly to suit the patient concerned, and secondly to suit different patient conditions.

The composition may be applied to the treatment area prior to placement of the apparatus as described hereinbefore. The apparatus can be provided separately from the composition or with the composition already contained within a drug delivery system. Alternatively the apparatus could be provided in the form of a kit comprising the apparatus and at least one composition comprising a photosensitiser or a precursor of a photosensitiser for use with the apparatus.

Hence viewed from another aspect the present invention provides a kit comprising the apparatus according to the invention and at least one composition comprising a photosensitiser or a precursor of a photosensitiser

The present apparatus and method for photodynamic treatment may be combined with other therapeutic procedures, for example administration of other therapeutic drugs. These therapeutic drugs might be administered into the body prior to or together with placing the apparatus to the treatment area or might be administered through other routes of administration (e.g. oral, intravascular or topical, e.g. dermal). Typically such drugs include antiviral agents, immune-modulating drugs or combination of such drugs.

Certain preferred embodiments of the present invention will now be described by way of example only.
Figure 1 shows an irradiation apparatus in the form of a pad for adhering to the patient's underwear;
Figure 2 shows a cross-section of the apparatus in schematic form;
Figure 3 shows an alternative arrangement in which the apparatus is included in a diaper type garment;
Figure 4 is a close up view of the crotch part of the diaper of Figure 3, illustrating the use of a removable layer; and
Figure 5 shows an irradiation apparatus using a pouch in the crotch of a garment in place of a removable layer.

As described herein the upper side of the apparatus is the side with the treatment surface, which generally faces upwards during use, and the lower side is the side opposite the treatment surface.

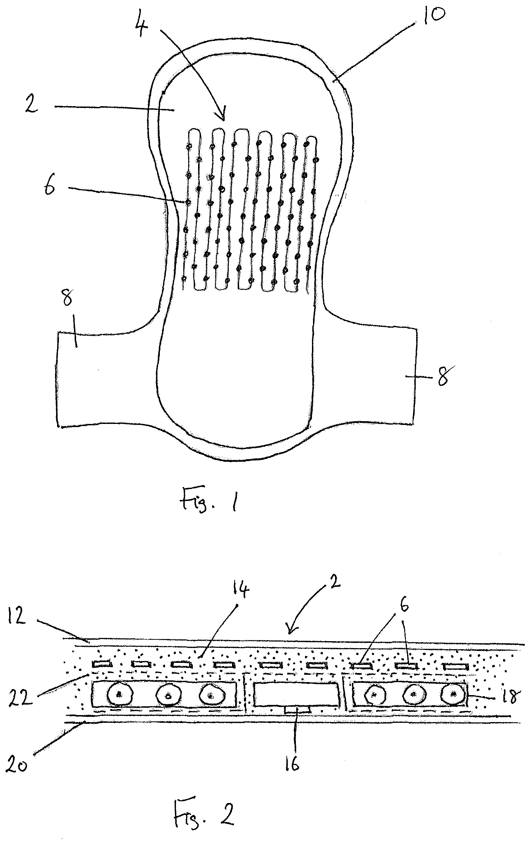

Figure 1 shows a first arrangement of the irradiation apparatus. The apparatus comprises a flat but somewhat flexible continuous upper treatment surface 2 in the form of a strip or sheet. The treatment surface 2 contains an illumination system 4 with LEDs 6 in strips of LEDs for projecting light from the treatment surface 2. The treatment surface 2 is arranged to confront a treatment area on the vulva and/or anus. In this example apparatus wings 8 are provided for coupling the apparatus to the patient's underwear. The wings 8 have an adhesive on the lower side so that they can be folded about the crotch part of the underwear and secured thereto. Alternatively to the position of the wings in Figure 1, said wings can also be provided at the front-end or at the front-end and back-end of the apparatus. The outer edge 10 of the apparatus can optionally include adhesive or a skin-adhering surface (for example, bioadhesive material) at the upper surface thereof in order to help the apparatus remain in place. Alternatively, the underside of the apparatus (not shown) can include an adhesive to help the apparatus to remain in place in a garment, e.g. a panty.

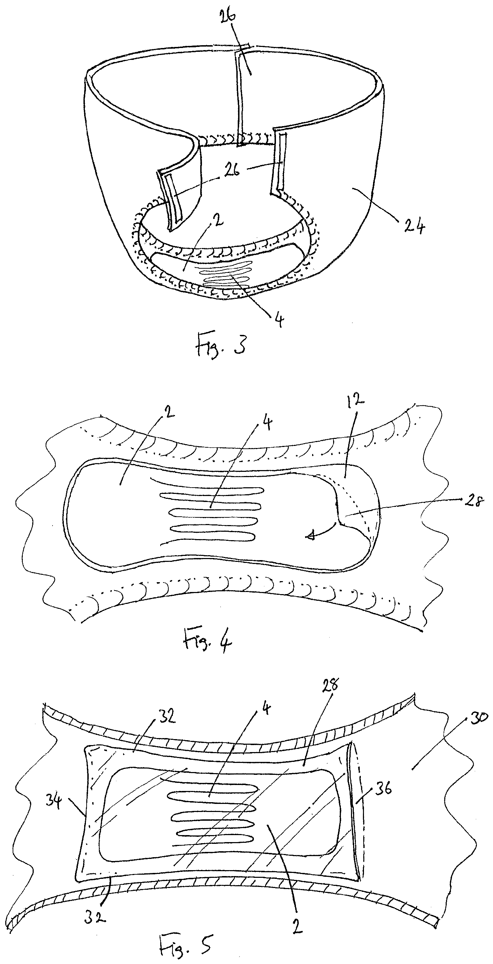

The internal structure of the apparatus is shown as a schematic cross-section in Figure 2. The apparatus has a layered construction. The upper layer is a polyurethane layer 12, which can be formed as an integral layer with other parts of the apparatus. Polyurethane is a suitable material for the upper layer 12 since it is water-tight and can easily be cleaned. Beneath the upper polyurethane layer 12 is a silicone layer 14 formed over and about the LEDs 6. The silicone layer 14 also encases other electrical parts of the apparatus. Depending on the type of silicone, the silicone layer may help to evenly distribute the light from the LEDs 6. White pigments are included in the silicon layer 14 to diffuse the light, provide a more even distribution of the light and increase the light dose due to back-scattering. On top of the silicon layer 14 and polyurethane layer 12, there is a polymer foil/film layer 28 (not shown in Figure 2, described below in relation to Figures 4 and 5) that can be removed and cleaned. This can be a removable and/or reusable layer as discussed in relation to Figures 4 and 5 below.

Thus, the apparatus has a treatment surface 2 formed of several parts, i.e. layers, including a substrate silicone layer 14 that holds the LEDs 6, a protecting polyurethane upper layer 12, and a removable/cleanable polymer film 28 on top of the polyurethane layer 12. The polymer film layer 28 can include a photosensitiser or precursor, e.g. in the form of a dry composition such as a dry film, preferably such dry compositions comprising 5-ALA or derivatives thereof as described in PCT/EP2011/061688 by Photocure ASA or as described by A. Zawislak et al. in Photodiagnosis and Photodynamic Therapy (2009), 6, 28-40).

The apparatus further includes a control circuit 16 and power source 18 for the LEDs 6. The control circuit 16 is encapsulated within the apparatus and may be encased in the silicone layer 14. It is enclosed by an additional flexible polyurethane layer 20 beneath/behind the silicon layer 14. The two polyurethane layers 12, 20 together form a housing and provide a seal for the electrical parts 6, 16, 18. They are joined at the outer edge 10 of the device to form a seal about the outer edge 10. A switch on the control circuit 16 is accessible through the lower flexible layer 20 for turning the apparatus on and off. The power source 18 comprises flat (coin type) batteries that are sealed within the apparatus between the two flexible layers 12, 20.

The apparatus optionally includes a heat exchanger 22 for removing heat from the treatment area. The heat exchanger includes thermally conducting parts adjacent to the treatment area in order to convey heat away from the patient.

Figure 3 shows an apparatus incorporated into a diaper type garment 24. The diaper 24 can be of any suitable construction. In the example of Figure 3 it includes an elasticated waist and elasticated legs with the waist including two fasteners 26 which would be placed at either side of the body when in use, as with a babies diaper or nappy. The fasteners 26 use hook and loop fasteners (e.g. Velcro®) in this example. The apparatus is fitted in the crotch part of the diaper 24 with the treatment surface 2 facing upward toward the body and the illumination system 4 placed to direct light on the required treatment area. Alternative designs can be provided for illumination of treatment areas on the vulva and/or anus, with the illumination system 4 included light sources placed at different locations within the diaper 24.

The crotch part of the diaper 24 is shown in an enlarged view in Figure 4 in order to show one arrangement for the polymer film layer 28. In this example it is a removable layer with adhesive properties, which can be positioned on top of the polyurethane layer 12 by the patient before use of the apparatus and peeled away from the polyurethane upper layer 12 by pulling in the direction of the arrow shown in the Figure after the treatment is finished. This polymer film layer 28 can hence be removed and discarded after the use of the apparatus for treatment of the patient, with a replacement polymer film layer 28 being used for a subsequent treatment. This type of replaceable layer 28 can be used with the apparatus of Figure 1 in a similar manner.

Figure 5 shows an enlarged view of the crotch part of a panty 30 with an alternative arrangement in which the removable foil layer 28 is attached to the crotch part of the panty 30 on the long sides 32 and one short side 34. This forms a pouch that allows the insertion of the other parts of the apparatus into the pouch and beneath the polymer foil layer 28 via the other short side 36, which has an opening. The treatment surface 2 and illumination system 4 are placed facing the foil layer 28 and hence will face the treatment area on the vulva and/or anus when the panty 30 is worn. The foil layer 28 in this arrangement is removable from the remainder of the apparatus by taking the apparatus out of the pouch. Thus, the majority of the parts of the apparatus, including the polyurethane layers 12, 20, power source 18, control circuit 16, illumination system 4 and silicon layer 14, are formed as a separate body which can be re-used with a new polymer foil layer 28. Alternatively the panty 30 comprises a non-removable polymer foil layer 28 which can be cleaned before re-use in a subsequent treatment.

The foil layer 28 in this arrangement may be provided separately to the panty 30 with suitable adhesive parts enabling it to be attached to an existing garment on the long sides 32 and one short side 34. Alternatively, the panty 30 is provided with the foil layer 28.

Although Figure 5 shows the pouch arrangement of the polymer foil layer 28 with a panty, it will be understood that it could equally well be used with a diaper 24 as in Figure 3.

Before the light is used the composition comprising a photosensitiser or precursor for the photodynamic therapy is applied to the patient. This is done either by applying it directly to the surface of the treatment area, or systematically by intravenously or orally administered compositions. The composition can be applied to the treatment surface 2 of the apparatus so that it is applied to the patient when the treatment surface 2 is placed on the treatment area.

The apparatus is applied to the vulva and/or anus with the treatment surface 2 facing the vulva and/or anus or, if present, the polymer film 28 in contact with the patient's body. The illumination system 4 is activated when the apparatus is turned on, or activated in accordance with a present timing set by the control circuit 16. Light is directed from the LEDs 6 through the silicone layer 14, protective polyurethane layer 12 and polymer film 28 and onto the desired treatment area at the desired fluence rate. The treatment time is controlled according to the programming of the control circuit 16, or it can also be set by a time of removal of the apparatus or by switching it off using the switch. As discussed above, the control circuit 16 for the illumination system 4 can optionally apply a pulsed illumination. After the treatment is complete the apparatus is removed. The apparatus could be single use, but in this preferred embodiment it is intended to be re-used. The polymer film 28 is cleaned or alternatively is removed and discarded with a new film being utilised for a subsequent use of the other parts of the apparatus.

The arrangements described herein are for illustration only and should not be taken to limit the scope of protection. The skilled man will appreciate that adjustments could be made without deviating from the scope of the claims. For example, other forms of control circuit and LED array/fibre optic array can be used. The wings 8 of Figure 1 may be placed more centrally on the apparatus. The polymer film layers 28 of Figures 4 and 5 can be used in combination with the features of any of Figures 1, 2 and 3 or in other alternatives as discussed above.

## Claims

1. A portable, self-contained, irradiation apparatus in the form of a diaper, panty liner, sanitary napkin, sanitary pad, or incontinence pad for photodynamic treatment of the vulva and/or the anus, the apparatus comprising: a treatment surface capable of conforming to the area of the vulva and/or anus to be treated, an illumination system for directing light onto a treatment area of the vulva and/or anus and a power source for the illumination system; wherein the illumination system is arranged to provide light at fluence rates of 50 mW/cm² or below; wherein the apparatus includes a flexible housing comprising two flexible layers sealed to one another about their periphery and a silicone layer located between the two flexible layers encasing light emitting parts of the illumination system; wherein one of the flexible layers forms a treatment surface; and wherein the power source and/or other electrical components is/are sealed within the housing.

2. An apparatus as claimed in claim 1, wherein the illumination system is arranged to apply illumination with a total light dose level of 10 to 200 J/cm²

3. An apparatus as claimed in claim 1 or 2, wherein the fluence rate is in the range of 0.5 to 40 mW/cm², preferably in the range of 2 to 20 mW/cm².

4. An apparatus as claimed in any preceding claim, wherein the illumination system is arranged to emit light having or consisting of wavelengths in the range of 300 to 800 nm.

5. An apparatus as claimed in claim 4, wherein the illumination system is arranged to emit light having or consisting of wavelengths in the range of 600 to 670 nm, most preferably light of about 630 nm.

6. An apparatus as claimed in any preceding claim, wherein the apparatus is capable of independent operation while the apparatus is applied to the vulva and/or anus and wherein the power source is integrated with the apparatus and arranged to be carried with the apparatus when the treatment surface is, in use, applied to the treatment area of the patient.

7. An apparatus as claimed in any preceding claim, wherein the treatment surface is sufficiently flexible to allow it to conform to the treatment area and thus maintain contact with the vulva and/or anus, when the apparatus is in use.

8. An apparatus as claimed in any preceding claim, wherein the treatment surface comprises a removable and/or cleanable layer as a top layer.

9. An apparatus as claimed in claim 8, wherein the removable and/or cleanable layer is connected to a garment to form a pouch, with the apparatus being insertable beneath the removable and/or cleanable layer to be contained within the pouch.

10. An apparatus as claimed in any preceding claim wherein the apparatus is arranged to be secured in place with the treatment surface located at the treatment area, when in use, such that the patient can continue with normal activities.

11. An apparatus as claimed in claim 10, wherein either the apparatus comprises a panty or diaper and the panty or diaper is arranged to hold the treatment surface at the treatment area when worn by the patient, or the apparatus includes a coupling for joining the apparatus to an undergarment such as a panty or diaper.

12. An apparatus as claimed in claim 11, including the coupling, wherein the coupling comprises an adhesive patch or patches for adhering the apparatus to a garment, for example an adhesive on an underside of the apparatus and/or an adhesive on wings provided at side portions of the apparatus.

13. An apparatus as claimed in any preceding claim, wherein the treatment surface has bioadhesive properties.

14. An apparatus as claimed in any preceding claim, comprising a drug delivery system for carrying a composition comprising a photosensitiser or precursor of a photosensitiser.

15. An apparatus as claimed in any preceding claim in combination with a composition comprising a photosensitiser or precursor of a photosensitiser.

16. An apparatus as claimed in claims 14 and 15, wherein the composition comprises a precursor of a photosensitiser, preferably 5-ALA or a derivative thereof.

17. An apparatus as claimed in claim 16 wherein the composition comprises a 5-ALA ester, preferably a 5-ALA ester of formula (I) and pharmaceutically acceptable salts thereof:
|R²₂N-CH₂COCH₂-CH₂CO-OR¹ (I)
wherein
R¹ represents a substituted or unsubstituted alkyl group; and
R² each independently represents a hydrogen atom or a group R¹.

18. A kit comprising an apparatus as claimed in any of claims 1 to 17 and at least one composition comprising a photosensitiser or precursor of a photosensitiser, preferably 5-ALA or a derivative thereof.

## Patentansprüche

1. Portable, eigenständige Bestrahlungseinrichtung in Form einer Windel, Slipeinlage, Monatsbinde, Damenbinde oder Inkontinenzeinlage zur photodynamischen Behandlung der Vulva und/oder des Anus, wobei die Einrichtung umfasst: eine Behandlungsoberfläche, die imstande ist, sich dem zu behandelnden Bereich der Vulva und/oder des Anus anzupassen, ein Beleuchtungssystem zum Lenken von Licht auf einen Behandlungsbereich der Vulva und/oder des Anus und eine Energiequelle für das Beleuchtungssystem;
wobei das Beleuchtungssystem angeordnet ist, um Licht mit Flussdichten von 50 mW/cm² oder darunter bereitzustellen; wobei die Einrichtung ein flexibles Gehäuse beinhaltet, das zwei flexible Schichten, die um ihre Peripherie miteinander versiegelt sind, und eine Silikonschicht umfasst, die sich zwischen den zwei flexiblen Schichten befindet, die lichtemittierende Teile des Beleuchtungssystems umschließen; wobei eine der flexiblen Schichten eine Behandlungsoberfläche bildet; und wobei die Energiequelle und/oder weitere elektrische Komponenten innerhalb des Gehäuses versiegelt ist/sind.

2. Einrichtung nach Anspruch 1, wobei das Beleuchtungssystem angeordnet ist, um Beleuchtung mit einem Gesamtlichtdosisniveau von 10 bis 200 J/cm² anzulegen.

3. Einrichtung nach Anspruch 1 oder 2, wobei die Flussdichte im Bereich von 0,5 bis 40 mW/cm², vorzugsweise im Bereich von 2 bis 20 mW/cm² liegt.

4. Einrichtung nach einem der vorstehenden Ansprüche, wobei das Beleuchtungssystem angeordnet ist, um Licht zu emittieren, das Wellenlängen im Bereich von 300 bis 800 nm aufweist oder daraus besteht.

5. Einrichtung nach Anspruch 4, wobei das Beleuchtungssystem angeordnet ist, um Licht zu emittieren, das Wellenlängen im Bereich von 600 bis 670 nm, am meisten bevorzugt Licht von etwa 630 nm, aufweist oder daraus besteht.

6. Einrichtung nach einem der vorstehenden Ansprüche, wobei die Einrichtung zu unabhängigem Betrieb imstande ist, während die Einrichtung an der Vulva und/oder dem Anus angelegt ist, und wobei die Energiequelle mit der Einrichtung integriert und angeordnet ist, um mit der Einrichtung getragen zu werden, wenn die Behandlungsoberfläche bei Gebrauch an dem Behandlungsbereich des Patienten angelegt ist.

7. Einrichtung nach einem der vorstehenden Ansprüche, wobei die Behandlungsoberfläche ausreichend flexibel ist, um ihr zu ermöglichen, sich an den Behandlungsbereich anzupassen, und somit den Kontakt mit der Vulva und/oder dem Anus beizubehalten, wenn die Einrichtung in Gebrauch ist.

8. Einrichtung nach einem der vorstehenden Ansprüche, wobei die Behandlungsoberfläche eine abnehmbare und/oder reinigbare Schicht als eine Oberschicht umfasst.

9. Einrichtung nach Anspruch 8, wobei die abnehmbare und/oder reinigbare Schicht mit einem Kleidungsstück verbunden ist, um einen Beutel zu bilden, wobei die Einrichtung unterhalb der abnehmbaren und/oder reinigbaren Schicht einsetzbar ist, um innerhalb des Beutels enthalten zu sein.

10. Einrichtung nach einem der vorstehenden Ansprüche, wobei die Einrichtung angeordnet ist, um, wenn in Gebrauch, mit der Behandlungsoberfläche an dem Behandlungsbereich lagegesichert zu sein, sodass der Patient mit normalen Tätigkeiten fortfahren kann.

11. Einrichtung nach Anspruch 10, wobei die Einrichtung entweder einen Slip oder eine Windel umfasst und der Slip oder die Windel angeordnet ist, um, wenn vom Patienten getragen, die Behandlungsoberfläche im Behandlungsbereich zu halten, oder die Einrichtung eine Kopplung zum Anfügen der Einrichtung an eine Unterwäsche, wie einem Slip oder einer Windel, beinhaltet.

12. Einrichtung nach Anspruch 11, die die Kopplung beinhaltet, wobei die Kopplung ein Klebepflaster oder Pflaster zum Kleben der Einrichtung an ein Kleidungsstück umfasst, beispielsweise einen Kleber an einer Unterseite der Einrichtung und/oder einen Kleber an Flügeln, die an Seitenabschnitten der Einrichtung bereitgestellt sind.

13. Einrichtung nach einem der vorstehenden Ansprüche, wobei die Behandlungsoberfläche bioadhäsive Eigenschaften aufweist.

14. Einrichtung nach einem der vorstehenden Ansprüche, umfassend ein Medikamentenabgabesystem zum Tragen einer Zusammensetzung, die einen Photosensibilisator oder Vorläufer eines Photosensibilisators umfasst.

15. Einrichtung nach einem der vorstehenden Ansprüche in Kombination mit einer Zusammensetzung, die einen Photosensibilisator oder Vorläufer eines Photosensibilisators umfasst.

16. Einrichtung nach Ansprüchen 14 und 15, wobei die Zusammensetzung einen Vorläufer eines Photosensibilisators, vorzugsweise 5-ALA oder ein Derivat davon, umfasst.

17. Einrichtung nach Anspruch 16, wobei die Zusammensetzung einen 5-ALA-Ester, vorzugsweise einen 5-ALA-Ester der Formel (I) und pharmazeutisch annehmbare Salze davon umfasst:
|R²₂N-CH₂COCH₂-CH₂CO-OR¹ (I)
wobei
R¹ eine substituierte oder nicht substituierte Alkylgruppe repräsentiert; und
R² jeweils unabhängig ein Wasserstoffatom oder eine Gruppe R¹ repräsentiert.

18. Kit, umfassend eine Einrichtung nach einem der Ansprüche 1 bis 17 und mindestens eine Zusammensetzung, umfassend einen Photosensibilisator oder Vorläufer eines Photosensibilisators, vorzugsweise 5-ALA oder ein Derivat davon.

## Revendications

1. Équipement d'irradiation autonome portable sous la forme d'une couche, d'un protège-slip, d'une serviette hygiénique, d'un tampon hygiénique ou d'un tampon pour incontinence pour un traitement photodynamique de la vulve et/ou de l'anus, l'équipement comprenant : un traitement de surface capable de se conformer à la zone de la vulve et/ou de l'anus à traiter, un système d'éclairage pour orienter la lumière sur une zone de traitement de la vulve et/ou de l'anus et une source d'alimentation pour le système d'éclairage ;
dans lequel le système d'éclairage est disposé pour fournir une lumière à des débits de fluence de 50 mW/cm² ou moins ; dans lequel l'équipement inclut un boîtier flexible comprenant deux couches flexibles scellées l'une à l'autre autour de leur périphérie et une couche de silicone située entre les deux couches flexibles encadrant des éléments électroluminescents du système d'éclairage ; dans lequel l'une des couches flexibles forme une surface de traitement ; et dans lequel la source d'alimentation et/ou d'autres composants électriques est/sont scellés dans le boîtier.

2. Équipement selon la revendication 1, dans lequel le système d'éclairage est disposé pour appliquer un éclairage avec un niveau de dose de lumière total de 10 à 200 J/cm².

3. Équipement selon la revendication 1 ou 2, dans lequel le débit de fluence est dans la plage de 0,5 à 40 mW/cm², de préférence dans la plage de 2 à 20 mW/cm².

4. Équipement selon une quelconque revendication précédente, dans lequel le système d'éclairage est disposé pour émettre une lumière présentant ou consistant en des longueurs d'onde dans la plage de 300 à 800 nm.

5. Équipement selon la revendication 4, dans lequel le système d'éclairage est disposé pour émettre une lumière présentant ou consistant en des longueurs d'onde dans la plage de 600 à 670 nm, le plus préférablement d'environ 630 nm.

6. Équipement selon une quelconque revendication précédente, dans lequel l'équipement est capable de fonctionnement indépendant tandis que l'équipement est appliqué sur la vulve et/ou l'anus et dans lequel la source d'alimentation est intégrée à l'équipement et disposée pour être portée avec l'équipement lorsque la surface de traitement est, pendant l'utilisation, appliquée sur la zone de traitement du patient.

7. Équipement selon une quelconque revendication précédente, dans lequel la surface de traitement est suffisamment flexible pour lui permettre de se conformer à la zone de traitement et ainsi maintenir un contact avec la vulve et/ou l'anus, lorsque l'équipement est en cours d'utilisation.

8. Équipement selon une quelconque revendication précédente, dans lequel la surface de traitement comprend une surface retirable et/ou nettoyable en tant que couche supérieure.

9. Équipement selon la revendication 8, dans lequel la couche retirable et/ou nettoyable est reliée à un vêtement pour former une poche, l'équipement étant insérable sous la couche retirable et/ou nettoyable devant être contenue dans la poche.

10. Équipement selon une quelconque revendication précédente, dans lequel l'équipement est disposé pour être maintenu en place avec la surface de traitement située au niveau de la zone de traitement, pendant l'utilisation, de telle sorte que le patient peut poursuivre ses activités normales.

11. Équipement selon la revendication 10, dans lequel soit l'équipement comprend une culotte ou une couche et la culotte ou la couche est disposée pour maintenir la surface de traitement au niveau de la zone de traitement lorsqu'elle est portée par le patient, soit l'équipement inclut un couplage pour assembler l'équipement sur un sous-vêtement tel qu'une culotte ou une couche.

12. Équipement selon la revendication 11, incluant le couplage, dans lequel le couplage comprend un ou des patches adhésifs pour faire adhérer l'équipement à un vêtement, par exemple un adhésif sur un dessous de l'équipement et/ou un adhésif sur des ailes prévues au niveau de parties latérales de l'équipement.

13. Équipement selon une quelconque revendication précédente, dans lequel la surface de traitement présente des propriétés bioadhésives.

14. Équipement selon une quelconque revendication précédente, comprenant un système d'administration de médicament pour transporter une composition comprenant un photosensibilisateur ou un précurseur d'un photosensibilisateur.

15. Équipement selon une quelconque revendication précédente, en combinaison avec une combinaison comprenant un photosensibilisateur ou un précurseur d'un photosensibilisateur.

16. Équipement selon les revendications 14 et 15, dans lequel la composition comprend un précurseur d'un photosensibilisateur, de préférence le 5-ALA ou un dérivé de celui-ci.

17. Équipement selon la revendication 16, dans lequel la composition comprend un ester de 5-ALA, de préférence un ester de 5-ALA de formule (I) et des sels pharmaceutiquement acceptables de celui-ci :
|R²₂N-CH₂COCH₂-CH₂CO-OR¹ (I)
dans lequel
R¹ représente un groupe alkyle substitué ou non substitué ; et
R² représente chaque fois indépendamment un atome d'hydrogène ou un groupe R¹.

18. Kit comprenant un équipement selon l'une quelconque des revendications 1 à 17 et au moins une composition comprenant un photosensibilisateur ou un précurseur d'un photosensibilisateur, de préférence le 5-ALA ou un dérivé de celui-ci.
